(19) Europäisches Patentamt
European Patent Office
Office européen des brevets

(11) EP 1 779 840 B1

(12) FASCICULE DE BREVET EUROPEEN

(45) Date de publication et mention de la délivrance du brevet:
**05.11.2008 Bulletin 2008/45**

(21) Numéro de dépôt: **06121556.2**

(22) Date de dépôt: **29.09.2006**

(51) Int Cl.:
***A61K 8/37*** *(2006.01)* ***A61K 8/73*** *(2006.01)*
***A61K 8/81*** *(2006.01)* ***A61Q 5/02*** *(2006.01)*
***A61Q 19/10*** *(2006.01)*

(54) **Compositions cosmétiques contenant un ester liquide, un polymère épaississant acrylique, une cyclodextrine et un tensioactif anionique, et leurs utilisations.**

Kosmetische Zusammensetzungen enthaltend einen flüssigen Ester, ein verdickendes Acrylpolymer, ein Cyclodextrin und ein anionisches Tensid, Verwendung dieser Zusammensetzungen

Cosmetic compositions comprising a liquid ester, an acrylic thickening polymer, a cyclodextrin and an anionic surfactant, and use thereof

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **26.10.2005 FR 0553256**

(43) Date de publication de la demande:
**02.05.2007 Bulletin 2007/18**

(73) Titulaire: **L'ORÉAL**
**75008 Paris (FR)**

(72) Inventeur: **Müller, Rainer**
**76344 Leopoldshafen (DE)**

(74) Mandataire: **Le Blainvaux Bellegarde, Françoise**
**L'OREAL - D.I.P.I.**
**25-29 Quai Aulagnier**
**92665 ASNIERES CEDEX (FR)**

(56) Documents cités:
**EP-A- 1 457 195** **WO-A-03/088934**

EP 1 779 840 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet des compositions cosmétiques (à usage topique) comprenant dans un milieu aqueux cosmétiquement acceptable au moins une cyclodextrine, au moins un tensioactif anionique, au moins un ester gras particulier et au moins un polymère épaississant acrylique. Elle a aussi pour objet des compositions comprenant en outre au moins un agent de conditionnement.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents de conditionnement, notamment insolubles, pour faciliter le démêlage des cheveux et pour leur communiquer douceur, brillance et souplesse.

Compte tenu du caractère insoluble de certains agents de conditionnement tels que par exemple les silicones ou les huiles, on cherche à maintenir les agents de conditionnement en dispersion régulière dans le milieu sans cependant faire chuter la viscosité et les propriétés détergentes et moussantes des compositions. Les agents de conditionnement notamment les silicones ou les huiles doivent également être véhiculés sur les matières kératiniques traitées en vue de leur conférer, suivant l'application, des propriétés de douceur, de brillance et de démêlage.

On sait également que les produits en particulier cosmétiques ayant un aspect ou un effet irisé, moiré ou métallisé, sont largement appréciés par les consommateurs pour leur aspect esthétique et donnant une apparence de richesse au produit. Les agents qui apportent cet effet sont des agents de nacrage ou agent nacrants comprenant généralement des cristaux qui restent dispersés dans les compositions et qui réfléchissent la lumière.
Les dérivés d'esters à longue chaîne sont largement utilisés pour nacrer les compositions notamment cosmétiques. Cependant, ces dérivés peuvent présenter des problèmes de cristallisation qui entraînent une évolution de la viscosité des compositions au cours du temps.

On connaît également les dérivés d'éthers ou de thioéthers à longue chaîne tels que ceux décrits dans les demandes EP457688 et WO98/03155. Cependant, ces derniers agents opacifient les compositions sans apporter de nacrage aux compositions ou pas suffisamment.

Il a été aussi constaté que ces agents nacrants, du fait de leur faible densité, présentaient souvent l'inconvénient de remonter à la surface du shampooing et d'y former une couche inesthétique pour le consommateur.

De plus ces composés à chaînes grasses présentent dans certains cas l'inconvénient d'apporter un toucher chargé aux cheveux, un manque de légèreté, de volume de la chevelure .
Par ailleurs, tous ces agents nacrants sont des composés insolubles dans l'eau et ont un point de fusion supérieur à 50°C. Pour fabriquer des compositions nacrées, il faut donc chauffer les compositions au-dessus du point de fusion du composé nacrant puis refroidir et ensuite ajouter les autres composés de la composition. Afin de diminuer la consommation d'énergie et de réduire la durée de fabrication, il est souhaitable de préparer les compositions à froid.

La demande WO03/088934 décrit l'utilisation de cyclodextrine comme agent nacrant qui peut être utilisé à froid. Cependant les compositions contenant une cyclodextrine ne sont pas encore suffisamment stables.

Pour épaissir et stabiliser les compositions cosmétiques contenant des agents conditionneurs insolubles, des agents de stabilisation tels que les polymères acryliques réticulés du type Carbopol sont fréquemment utilisés. Néanmoins, ces agents de stabilisation ne sont pas toujours suffisant pour assurer une bonne stabilité au stockage dans le temps, notamment à température élevée(45°C)

La demanderesse a découvert qu'il était possible de formuler des compositions cosmétique pour le traitement des matières kératiniques, en particulier des shampooings présentant un aspect nacré tout en ayant les propriétés esthétiques et cosmétiques recherchées, en utilisant dans ces compositions cosmétiques au moins une cyclodextrine ou l'un de ses dérivés, au moins un tensioactif, au moins un ester gras particulier et au moins un polymère acrylique.

L'invention a pour objet des compositions notamment cosmétiques comprenant dans un milieu aqueux notamment physiologiquement acceptable et en particulier cosmétiquement acceptable au moins un agent tensioactif anionique, au moins un ester liquide d'acide carboxylique ayant de 4 à 6 atomes de carbone et d'alcool ayant de 12 à 26 atomes de carbone, au moins une cyclodextrine ou l'un de ses dérivés et au moins un polymère épaississant acrylique (à définir).

Les compositions présentent une très bonne homogénéité et une bonne stabilité du nacrage, ainsi qu'une viscosité satisfaisante pour l'application sur les matières kératiniques. Il n'y a pas de déphasage (séparation de phase) au cours du temps de la cyclodextrine et/ou des agents de conditionnement insolubles.

En particulier, il ne se produit aucun relargage ou épaississement incontrôlé de la composition au cours du temps. Les compositions présentent enfin une texture non filante et fondante. La mousse se rince facilement.

Un autre objet de l'invention est constitué par le procédé de lavage et/ou de conditionnement mettant en oeuvre de telles compositions.

Un autre objet de l'invention concerne un procédé pour améliorer la stabilité au stockage d'une composition

cosmétique comprenant au moins une cyclodextrine et au moins un tensioactif anionique consistant à associer dans ladite composition un polymère acrylique et un ester liquide d'acide carboxylique ayant de 4 à 6 atomes de carbone et d'alcool ayant de 12 à 26 atomes de carbone.

D'autres objets de l'invention apparaîtront à la lecture de la description et des exemples qui suivent.

Selon la présente invention, par matières kératiniques, on comprend les cheveux, les cils, les sourcils, la peau, les ongles, les muqueuses ou le cuir chevelu et plus particulièrement les cheveux.

Par le terme "agent de nacrage" ou "agent nacrant", on entend un agent produisant un aspect ou un effet nacré, irisé, moiré ou métallisé.

Les compositions de l'invention comprennent au moins un polymère épaississant acrylique.

Par polymère acrylique on entend au sens de la présente invention un polymère résultant de la polymérisation d'au moins un ou plusieurs monomères de structure

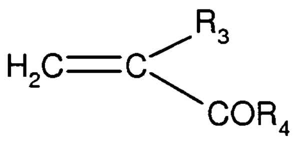

R3 désignant un atome d'hydrogène , un radical alkyl en C1-C4 linéaire ou ramifié, R4 désignant un atome d'hydrogène , un radical alkyl en C1-C4 linéaire ou ramifié, un radical NR5R6, un radical alcoxy en C1-C30 linéaire ou ramifi, éventuellement substitué par un ou plusieurs radicaux hydroxyle, par un radical ammonium quaternaire

R5 et R6 désignent un atome d'hydrogène , un radical alkyl en C1-C30 éventuellement oxyalkyléné, le radical alkyle pouvant comporter un groupement sulfonique.

De préférence R3 désigne un atome d'hydrogène ou un radical méthyle.

Par polymère épaississant, on entend au sens de la présente invention un polymère présentant en solution ou en dispersion à 1% en poids de matière active dans l'eau ou dans l'éthanol à 25°C, une viscosité supérieure à 0,2 Poise à un taux de cisaillement de 1 $s^{-1}$. La viscosité est mesurée avec un viscosimètre HAAKE RS600 de THERMO ELECTRON. Ce viscosimètre est un viscosimètre à contrainte imposée en géométrie cône plan (par exemple de diamètre 60 mm)

Les polymères épaississants sont notamment choisis parmi :

(a) les épaississants associatifs acryliques;
(b) les homopolymères d'acide acrylique réticulés ;
(c) les copolymères réticulés d'acide (méth)acrylique et d'acrylate d'alkyle($C_1$-$C_6$)
(d) les homopolymères et copolymères non-ioniques contenant des monomères à insaturation éthylénique de type ester et/ou amide ;
(e) les homopolymères d'acrylate d'ammonium ou les copolymères d'acrylate d'ammonium et d'acrylamide ;
(f) les homopolymères et copolymères d'acides (méth)acrylamido alkyl(C1-C4) sulfoniques
(g) les homopolymères et copolymères de méthacryoyl alkyl(C1-C4) trialkyl(C1-C4) ammonium réticulés

Par l'expression "épaississant associatif", on entend selon l'invention, un épaississant amphiphile comportant à la fois des motifs hydrophiles et des motifs hydrophobes en particulier comportant au moins une chaîne grasse en C8-C30 et au moins un motif hydrophile.

a) On peut utiliser comme épaississants associatifs acryliques selon l'invention les polymères associatifs acryliques choisis parmi :

(i) les polymères amphiphiles non-ioniques comportant au moins une chaîne grasse et au moins un motif hydrophile ;
(ii) les polymères amphiphiles anioniques comportant au moins un motif hydrophile, et au moins un motif à chaîne grasse ;
(iii) les polymères amphiphiles cationiques comportant au moins un motif hydrophile, et au moins un motif à chaîne grasse ;
(iv) les polymères amphiphiles amphotères comportant au moins un motif hydrophile, et au moins un motif à chaîne grasse ; les chaînes grasses ayant de 10 à 30 atomes de carbone.

**(i)** Les polymères amphiphiles non-ioniques acryliques comportant au moins une chaîne grasse et au moins

un motif hydrophile sont choisis de préférence parmi :

**(1)** les copolymères de méthacrylates ou d'acrylates d'alkyles en $C_1$-$C_6$ et de monomères amphiphiles comportant au moins une chaîne grasse (par exemple les acrylates d'alkyl (C8-C22) oxyéthyléné) tels que par exemple le copolymère méthacrylate de méthyle/acrylate de stéaryle oxyéthyléné vendu par la société GOLDSCHMIDT sous la dénomination ANTIL 208.
**(2)** les copolymères de méthacrylates ou d'acrylates hydrophiles et de monomères hydrophobes comportant au moins une chaîne grasse (par exemple les (meth)acrylates d'alkyl (C8-C22)) tels que par exemple le copolymère méthacrylate de polyéthylèneglycol/méthacrylate de lauryle.

**(ii)** Les polymères amphiphiles anioniques acryliques peuvent être choisis parmi ceux comportant au moins un motif hydrophile de type acide carboxylique insaturé oléfinique, et au moins un motif hydrophobe de type ester d'alkyl ($C_{10}$-$C_{30}$) d'acide carboxylique insaturé. Ils sont choisis de préférence parmi ceux dont le motif hydrophile de type acide carboxylique insaturé oléfinique correspond au monomère de formule (IV) suivante :

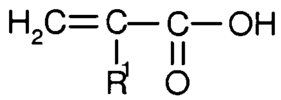

(IV)

formule dans laquelle, $R^1$ désigne H ou $CH_3$ ou $C_2H_5$, c'est-à-dire des motifs acide acrylique, acide méthacrylique ou acide éthacrylique et dont le motif hydrophobe de type ester d'alkyl ($C_{10}$-$C_{30}$) d'acide carboxylique insaturé correspond au monomère de formule (V) suivante :

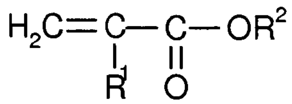

(V)

formule dans laquelle, $R^1$ désigne H ou $CH_3$ ou $C_2H_5$ (c'est-à-dire des motifs acrylates, méthacrylates ou éthacrylates) et de préférence H (motifs acrylates) ou $CH_3$ (motifs méthacrylates), $R^2$ désignant un radical alkyle en $C_{10}$-$C_{30}$, et de préférence en $C_{12}$-$C_{22}$.
Des esters d'alkyls ($C_{10}$-$C_{30}$) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
Des polymères amphiphiles anioniques de ce type sont par exemple décrits et préparés, selon les brevets US-3 915 921 et 4 509 949.
Les polymères amphiphiles anioniques utilisables dans le cadre de la présente invention peuvent désigner plus particulièrement des polymères formés à partir d'un mélange de monomères comprenant :

(i) de l'acide acrylique et un ou plusieurs esters de formule (VI) suivante :

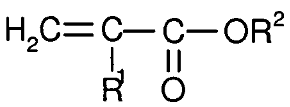

(VI)

dans laquelle $R^1$ désigne H ou $CH_3$, $R^2$ désignant un radical alkyle ayant de 12 à 22 atomes de carbone, et un agent réticulant, tels que par exemple ceux constitués de 95 à 60% en poids d'acide acrylique (motif hydrophile), 4 à 40% en poids d'acrylate d'alkyles en $C_{10}$-$C_{30}$ (motif hydrophobe), et 0 à 6% en poids de monomère polymérisable réticulant, ou 98 à 96% en poids d'acide acrylique (motif hydrophile), 1 à 4% en poids d'acrylate d'alkyles en $C_{10}$-$C_{30}$ (motif hydrophobe), et 0,1 à 0,6% en poids de monomère polymérisable réticulant,
(ii) essentiellement de l'acide acrylique et du méthacrylate de lauryle tel que celui formé à partir de 66% en poids d'acide acrylique et 34% en poids de méthacrylate de lauryle.

Ledit réticulant est un monomère contenant un groupe

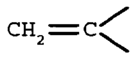

avec au moins un autre groupement polymérisable dont les liaisons insaturées sont non conjuguées l'une par rapport à l'autre. On peut notamment citer les polyallyléthers tels que notamment le polyallylsucrose et le polyallylpentaérythritol.

Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.C. sous la dénomination COATEX SX.

Comme polymères amphiphiles anioniques à chaînes grasses, on peut également citer le copolymère acide méthacrylique/acrylate de méthyle/diméthylmétaisopropényl benzyl isocyanate d'alcool éthoxylé commercialisé sous la dénomination VISCOPHOBE DB 1000 par la société AMERCHOL.

Comme autres polymères amphiphiles anioniques à chaînes grasses, on peut citer ceux comportant au moins un monomère acrylique à groupement(s) sulfonique(s), sous forme libre ou partiellement ou totalement neutralisée et comprenant au moins une partie hydrophobe.

La partie hydrophobe présente dans les polymères de l'invention comporte de préférence de 8 à 22 atomes de carbone, plus préférentiellement encore de 8 à 18 atomes de carbone, et plus particulièrement de 12 à 18 atomes de carbone.

De façon préférentielle, les polymères sulfoniques conformes à l'invention sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Les polymères amphiphiles sulfoniques conformes à l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Les polymères amphiphiles sulfoniques selon l'invention peuvent être réticulés ou non-réticulés. De préférence, on choisit des polymères amphiphiles réticulés.

Lorsqu'ils sont réticulés, les agents de réticulation peuvent être choisis parmi les composés à polyinsaturation oléfinique couramment utilisés pour la réticulation des polymères obtenus par polymérisation radicalaire. On peut citer par exemple, le divinylbenzène, l'éther diallylique, le dipropylèneglycol-diallyléther, les polyglycol-diallyléthers, le triéthylèneglycol-divinyléther, l'hydroquinone-diallyléther, le diacrylatedi(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilisera plus particulièrement le méthylène-bis-acrylamide, le méthacrylate d'allyle ou le triméthylol propane triacrylate (TMPTA). Le taux de réticulation variera en général de 0,01 à 10% en mole et plus particulièrement de 0,2 à 2% en mole par rapport au polymère.

Les monomères acryliques à groupement(s) sulfonique(s) sont choisis notamment parmi les acides (méth) acrylamido($C_1$-$C_{22}$)alkylsulfoniques, les acides N-($C_1$-$C_{22}$)alkyl(méth)acrylamido($C_1$-$C_{22}$)alkylsulfoniques comme l'acide undécylacrylamido-méthane-sulfonique ainsi que leurs formes partiellement ou totalement neutralisées.

Plus préférentiellement, on utilisera les acides (méth)acrylamido($C_1$-$C_{22}$) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

Plus particulièrement, on utilisera l'acide 2-acrylamido-2-méthylpropane-sulfonique (AMPS) ainsi que ses formes partiellement ou totalement neutralisées.

Les polymères amphiphiles conformes à l'invention peuvent notamment être choisis parmi les polymères amphiphiles statistiques d'AMPS modifiés par réaction avec une n-monoalkylamine ou une di-n-alkylamine en $C_6$-$C_{22}$, et tels que ceux décrits dans la demande de brevet WO00/31154 (faisant partie intégrante du contenu

de la description). Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.
Les polymères préférés de l'invention sont choisis parmi les copolymères amphiphiles d'AMPS et d'au moins un monomère hydrophobe à insaturation éthylénique comportant au moins une partie hydrophobe ayant de 8 à 50 atomes de carbone et plus préférentiellement de 8 à 22 atomes de carbone et encore plus préférentiellement de 8 à 18 atomes de carbone et plus particulièrement 12 à 18 atomes de carbone.
Ces mêmes copolymères peuvent contenir en outre un ou plusieurs monomères éthyléniquement insaturés ne comportant pas de chaîne grasse tels que les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.
Ces copolymères sont décrits notamment dans la demande de brevet EP-A-750899, le brevet US 5089578 et dans les publications de Yotaro Morishima suivantes :

- « Self-assembling amphiphilic polyelectrolytes and their nanostructures - Chinese Journal of Polymer Science Vol. 18, N °40, (2000), 323-336. »

- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N°10-3694-3704 »;

- « Solution properties of miscelle networks formed by non-ionic moieties covalently bound to an polyelectrolyte : salt effects on rheological behavior - Langmuir, 2000, Vol.16, N°12, 5324-5332 » ;

- « Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

Les monomères hydrophobes à insaturation éthylénique de ces copolymères particuliers sont choisis de préférence parmi les acrylates ou les acrylamides de formule (I) suivante :

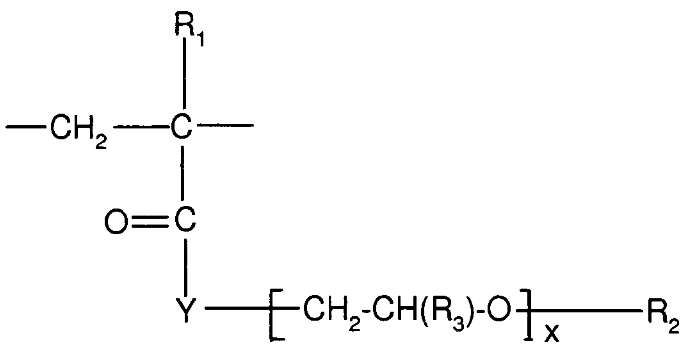

(I)

dans laquelle $R_1$ et $R_3$, identiques ou différents, désignent un atome d'hydrogène ou un radical alkyle linéaire ou ramifié en $C_1$-$C_6$ (de préférence méthyle) ; Y désigne O ou NH ; $R_2$ désigne un radical hydrocarboné hydrophobe comportant au moins de 8 à 50 atomes de carbone et plus préférentiellement de 8 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 0 à 100.
Le radical $R_2$ est choisi de préférence parmi les radicaux alkyles en $C_6$-$C_{18}$ linéaires (par exemple n-hexyle, n-octyle, n-décyle, n-hexadécyle, n-dodécyle), ramifiés ou cycliques (par exemple cyclododécane ($C_{12}$) ou adamantane ($C_{10}$)) ; les radicaux alkylperfluorés en $C_6$-$C_{18}$ (par exemple le groupement de formule -$(CH_2)_2$-$(CF_2)_9$-$CF_3$) ; le radical cholestéryle ($C_{27}$) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.
Selon une forme particulièrement préférée de l'invention, le monomère de formule (I) comporte au moins un motif oxyde d'alkylène (x ≥1) et de préférence une chaîne polyoxyalkylénée. La chaîne polyoxyalkylénée, de

façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 7 à 25.
Parmi ces polymères, on peut citer :

- les copolymères réticulés ou non réticulés, neutralisés ou non, comportant de 15 à 60% en poids de motifs AMPS et de 40 à 85% en poids de motifs ($C_8$-$C_{16}$)alkyl(méth)acrylamide ou de motifs ($C_8$-$C_{16}$)alkyl(méth) acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de motifs n-($C_6$-$C_{18}$)alkylacrylamide, tels que ceux décrits dans le brevet US-5089578.

On peut également citer les copolymères d'AMPS totalement neutralisé et de méthacrylate de dodécyle ainsi que les copolymères d'AMPS et de n-dodécylméthacrylamide non-réticulés et réticulés, tels que ceux décrits dans les articles de Morishima cités ci-dessus.
On citera plus particulièrement les copolymères constitués de motifs acide 2-acrylamido-2-méthylpropane-sulfonique (AMPS) de formule (II) suivante :

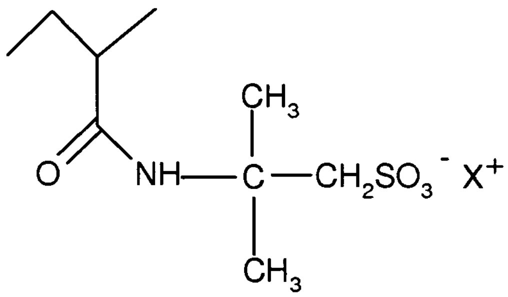

(II)

dans laquelle $X^+$ est un proton, un cation de métal alcalin, un cation alcalino-terreux ou l'ion ammonium,
et de motifs de formule (III) suivante :

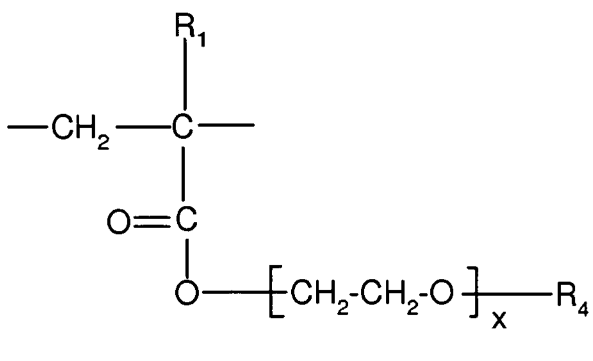

(III)

dans laquelle x désigne un nombre entier variant de 3 à 100, de préférence de 5 à 80 et
plus préférentiellement de 7 à 25 ; $R_1$ a la même signification que celle indiquée ci-dessus dans la formule (I) et $R_4$ désigne un alkyle linéaire ou ramifié en $C_6$-$C_{22}$ et plus préférentiellement en $C_{10}$-$C_{22}$.
Les polymères particulièrement préférés sont ceux pour lesquels x = 25 , $R_1$ désigne méthyle et $R_4$ représente n-dodécyle ; ils sont décrits dans les articles de Morishima mentionnés ci-dessus.
Les polymères pour lesquels $X^+$ désigne sodium ou ammonium sont plus particulièrement préférés.

**(iii)** Les polymères amphiphiles cationiques utilisés dans la présente invention sont choisis de préférence parmi les polyacrylates à groupements latéraux aminés.
Les polyacrylates à groupements latéraux aminés, quaternisés ou non, possèdent par exemple des groupements hydrophobes du type stéareth-20 (alcool stéarylique polyoxyéthyléné(20)) ou alkyl(C10-C30)PEG-20 itaconate. Comme exemples de polyacrylates à chaînes latérales aminées, on peut citer les polymères 8781-124B ou 9492-103 ou STRUCTURE PLUS de la société NATIONAL STARCH.

**(iv)** A titre de polymères amphiphiles amphotère contenant au moins une chaîne grasse, on peut citer les copolymères de chlorure de méthacrylamidopropyl triméthylammonium/acide acrylique/méthacrylate d'alkyle en C10-C30, le radical alkyle étant de préférence un radical stéaryle.

**(b)** Parmi les homopolymères d'acide acrylique réticulés, on peut citer ceux réticulés par un éther allylique d'alcool de la série du sucre, comme par exemple les produits vendus sous les noms CARBOPOLS 980, 981, 954, 2984 et 5984 par la société GOODRICH ou les produits vendus sous les noms SYNTHALEN M et SYNTHALEN K par la société 3 VSA.

**(c)** Parmi les copolymères réticulés d'acide (méth)acrylique et d'acrylate d'alkyle en $C_1$-$C_6$, on peut citer le produit vendu sous la dénomination VISCOATEX 538C par la société COATEX qui est un copolymère réticulé d'acide méthacrylique et d'acrylate d'éthyle en dispersion aqueuse à 38% de Matière Active ou le produit vendu sous la dénomination ACULYN 33 par la société ROHM & HAAS qui est un copolymère réticulé d'acide acrylique et d'acrylate d'éthyle en dispersion aqueuse à 28% de Matière Active. On peut citer plus particulièrement le copolymère acide méthacrylique/acrylate d'éthyle réticulé sous forme de dispersion aqueuse à 30% fabriqué et vendu sous le nom CARBOPOL AQUA SF-1 par la société NOVEON.

**(d)** Parmi les homopolymères ou copolymères non-ioniques contenant des monomères à insaturation éthylénique de type ester et/ou amide, on peut citer les produits vendus sous les dénominations de : CYANAMER P250 par la société CYTEC (polyacrylamide); PMMA MBX-8C par la société US COSMETICS (copolymère méthacrylate de méthyle / diméthacrylate d'éthylèneglycol); ACRYLOID B66 par la société RHOM & HAAS (copolymère méthacrylate de butyle / méthacrylate de méthyle); BPA 500 par la société KOBO (polyméthacrylate de méthyle).

**(e)** Parmi les homopolymères d'acrylate d'ammonium, on peut citer le produit vendu sous le nom MICROSAP PAS 5193 par la société HOECHST.
Parmi les copolymères d'acrylate d'ammonium et d'acrylamide, on peut citer le produit vendu sous le nom BOZEPOL C NOUVEAU ou le produit PAS 5193 vendus par la société HOECHST (ils sont décrits et préparés dans les documents FR-2416723, US-2798053 et US-2923692).

**(f)** Acides poly(méth)acrylamido-alkyl($C_1$-$C_4$)-sulfoniques
Selon la présente invention, le ou les acides poly(méth)acrylamido-alkyl($C_1$-$C_4$)-sulfoniques sont de préférence réticulés.
Plus particulièrement encore, ils sont partiellement ou totalement neutralisés.
Ce sont des polymères hydrosolubles ou gonflables dans l'eau.
Parmi ces polymères, on peut citer notamment :

- l'acide polyacrylamido-méthane-sulfonique,
- l'acide polyacrylamido-éthane-sulfonique,
- l'acide polyacrylamido-propane-sulfonique,
- l'acide poly-2-acrylamido-2-méthylpropane-sulfonique,
- l'acide poly-2-méthacrylamido-2-méthylpropane-sulfonique,
- l'acide poly-2-acrylamido-n-butane-sulfonique.

Des polymères de ce type et notamment des acides poly-2-acrylamido-2-méthylpropane-sulfoniques réticulés et partiellement ou totalement neutralisés sont connus, décrits et préparés dans la demande de brevet DE-19625810. Ils sont en général caractérisés par le fait qu'ils comprennent, distribués de façon aléatoire :

a) de 90 à 99,9% en poids de motifs de formule (I) suivante :

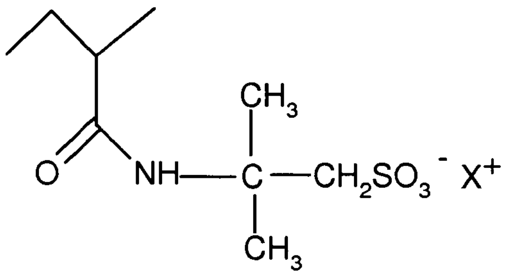

**(I)**

dans laquelle $X^+$ désigne un cation ou un mélange de cations, dont $H^+$,

b) de 0,01 à 10% en poids d'au moins un motif réticulant ayant au moins deux doubles-liaisons oléfiniques, les proportions en poids étant définies par rapport au poids total du polymère;

$X^+$ représente un cation ou un mélange de cations choisis en particulier parmi un proton, un cation de métal alcalin, un cation équivalent de celui d'un métal alcalino-terreux ou l'ion ammonium.

De préférence, l'acide poly-2-acrylamido-2-méthylpropane-sulfonique réticulé et neutralisé comporte de 98 à 99,5 % en poids de motifs de formule (I) et de 0,5 à 2 % en poids de motifs réticulants.

Les motifs réticulants ayant au moins deux doubles-liaisons oléfiniques sont choisis par exemple parmi le dipropylèneglycol-diallyléther, les polyglycol-diallyléthers, le triéthylèneglycol-divinyléther, l'hydroquinone-diallyl-éther, le tétra-allyl-oxyéthane ou d'autres allyl- ou vinyl-éthers d'alcools polyfonctionnels, le diacrylate de tétraéthylèneglycol, la triallylamine, le triméthylolpropane-diallyléther, le méthylène-bis-acrylamide ou le divinylbenzène.

Les motifs réticulants ayant au moins deux doubles-liaisons oléfiniques sont plus particulièrement encore choisis parmi ceux répondant à la formule générale (II) suivante :

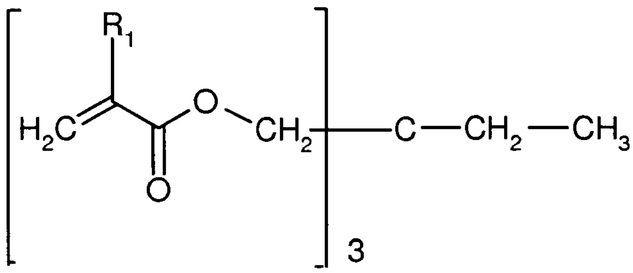

**(II)**

dans laquelle $R_1$ désigne un atome d'hydrogène ou un alkyle en $C_1$-$C_4$ et plus particulièrement méthyle (triméthylol propane triacrylate).

Les acides poly-2-acrylamido-2-méthylpropane-sulfoniques réticulés et partiellement ou totalement neutralisés sont généralement connus sous les appellations "Ammonium Polycrylamido-2-methylpropanesulfonate" ou encore "Ammonium Polyacryldimethyltauramide" (appellation I.N.C.I.).

Un produit particulièrement préféré selon l'invention est celui vendu par la société CLARIANT sous la dénomination commerciale HOSTACERIN AMPS; c'est un acide poly-2-acrylamido-2-méthylpropane sulfonique réticulé et partiellement neutralisé par l'ammoniaque.

**(g)** Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société CIBA.

Selon l'invention, le ou les polymère épaississant acrylique peuvent représenter de 0,001 % à 20 % en poids, de préférence de 0,01 % à 10% en poids et plus particulièrement de 0,1 à 3% en poids par rapport au poids total de la composition finale.

Les compositions selon l'invention comprennent nécessairement au moins un ester liquide d'acide carboxylique ayant de 4 à 6 atomes de carbone et d'alcool ayant de 12 à 26 atomes de carbone. Ces esters sont insolubles dans l'eau à une concentration supérieure à 0,1% à 25°C. Ils sont liquides à 25°C (1atm).

Les esters liquides selon l'invention ont de préférence la formule suivante :

$R_1\ COOR_2$ (I)

dans laquelle :

$R_1$ désigne un radical hydrocarboné, linéaire ou ramifié, éventuellement mono ou polyhydroxylé, ayant de 3 à 5 atomes de carbone,

$R_2$ désigne un radical hydrocarboné, linéaire ou ramifié, éventuellement mono ou polyhydroxylé, ayant de 12 à 26 atomes de carbone, de préférence ayant de 16 à 22 atomes de carbone,

$R_1$ désigne de préférence un radical alkyle ramifié ayant de 3 à 5 atomes de carbone, et plus particulièrement un radical tertio-butyle.

$R_2$ désigne de préférence un radical alkyle saturé ou insaturé ayant 12 à 26 atomes de carbone, plus particulièrement ramifié et encore plus particulièrement choisi parmi les radicaux tridécyle, isocétyle, isostéaryle, octyldodécyle et isoarachidyle.

Les esters liquides particulièrement préférés sont le néopentanoate d'isostéaryle (formule (I) dans laquelle $R_1$=tertio-butyle et $R_2$=isostéaryle), le néopentanoate de tridécyle, le néopentanoate d'isocétyle, et le néopentanoate d'isoarachidyle.

Le ou les esters liquides définis ci-dessus peuvent être utilisées dans les compositions conformes à l'invention dans des concentrations généralement comprises entre 0,1 et 20 %, et de préférence entre 0,2 et 10 % en poids par rapport au poids total de la composition et encore plus particulièrement de 0,5 à 5% en poids.

Les cyclodextrines sont notamment des oligosaccharides de formule :

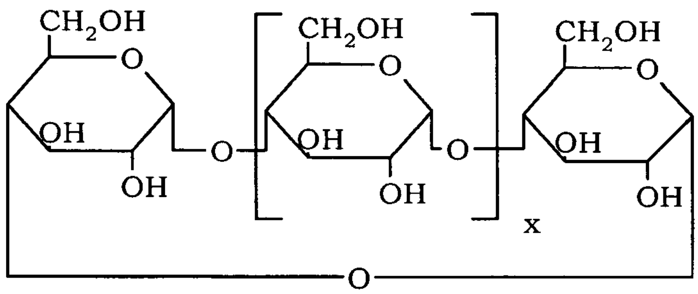

dans laquelle x peut être un nombre égal à 4 (ce qui correspond à l'α-cyclodextrine), à 5 (β-cyclodextrine) ou à 6 (γ-cyclodextrine).

On peut notamment utiliser une beta-cyclodextrine vendu par la société WACKER sous la dénomination CAVAMAX W7 et une gamma cyclodextrine vendu par la société WACKER sous la dénomination CAVAMAX W8.

Les dérivés de cyclodextrines sont par exemple les méthyl cyclodextrines tels que la méthyl-beta-cyclodextrine commercialisée par la société WACKER sous la dénomination CAVASOL W7M, les hydroxypropyl cyclodextrines telles que par exemple l'hydroxypropyl beta cyclodextrine commercialisé sous la dénomination CAVASOL W7 HP par la société WACKER.

Selon l'invention, la ou les cyclodextrines peuvent représenter de 1 à 15 % en poids, de préférence de 1 % à 10 % en poids, et encore plus préférentiellement de 1,5 % à 5 % en poids, du poids total de la composition finale.

Les compositions de l'invention comprennent en outre au moins un tensioactif anionique qui est présent de préférence en une quantité allant de 4% à 35% et encore plus préférentiellement de 8 % à 30%, par rapport au poids total de la composition.

La cyclodextrine ou l'un de ses dérivés et le tensioactif sont présents de préférence à une concentration efficace pour nacrer la composition et/ou pour former un complexe insoluble dans la composition entre la cyclodextrine et le tensioactif ou les tensioactifs.

De préférence, la cyclodextrine est introduite dans la composition sous forme non complexée ou éventuellement complexée avec le tensioactif ou les tensioactifs, c'est-à-dire que lorque qu'un complex est formé, il n'a pas été formé avec un autre composé que les tensioactifs.

Dans la composition, la cyclodextrine est complexée avec le ou les tensioactifs.

Le rapport tensioactif/cyclodextrine peut varier de 0,01 à 300, de préférence de 0,1 à 100 et plus particulièrement de 0,3 à 25.

La nature du tensioactif anionique ne revêt pas, dans le cadre de la présente invention, de caractère véritablement critique.

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkyl sulfates, les alkyl éther sulfates, alkyl amido éther sulfates, alkyl aryl polyéther sulfates, monoglycérides sulfates ; les alkyl sulfonates, alkylphosphates, alkyl amido sulfonates, alkyl aryl sulfonates, $\alpha$-oléfine-sulfonates, paraffine-sulfonates ; les alkyl sulfo succinates, les alkyl éther sulfosuccinates, les alkyl amide sulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl ($C_8$-$C_{24}$) éther carboxyliques polyoxyalkylénés, les acides alkyl($C_8$-$C_{24}$)aryl éther carboxyliques polyoxyalkylénés, les acides alkylamido($C_8$-$C_{24}$) éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

La composition peut contenir en plus du tensioactif anioniques des tensioactifs choisis parmi les tensioactifs non ioniques, amphotères et cationiques. De préférence les tensioactifs additionnels sont amphotères et/ non ioniques.

<u>Tensioactif(s) non ionique(s)</u> :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools polyéthoxylés, polypropoxylés ou polyglycérolés, les alpha-diols polyéthoxylés, polypropoxylés ou polyglycérolés, les alkylphénols polyéthoxylés, polypropoxylés ou polyglycérolés ou les acides gras polyéthoxylés, polypropoxylés ou polyglycérolés, tous ces composés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras de polyéthylèneglycol, les alkylpolyglycosides, les dérivés de N-alkyl glucamine, les oxydes d'amines tels que les oxydes d'alkyl ($C_{10}$-$C_{14}$) amines ou les oxydes de N-acylaminopropylmorpholine. On notera que les alkylpolyglycosides constituent des tensioactifs non-ioniques rentrant particulièrement bien dans le cadre de la présente invention.

<u>Tensioactif(s) amphotère(s) ou zwittérionique(s)</u> :

Les agents tensioactifs amphotères ou zwittérioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits commercialisés sous la dénomination MIRANOL, tels que décrits dans les brevets US-2528378 et US-2781354 et classés dans le dictionnaire CTFA, 3ème édition, 1982, sous les dénominations Amphocarboxy-glycinates et Amphocarboxypropionates de structures respectives :

$$R_2\text{ -CONHCH}_2\text{CH}_2\text{ -N}(R_3)(R_4)(\text{CH}_2\text{COO-}) \qquad (2)$$

dans laquelle : $R_2$ désigne un radical alkyle d'un acide $R_2$-COOH présent dans l'huile de coprah hydrolysée, un radical

heptyle, nonyle ou undécyle, $R_3$ désigne un groupement bêta-hydroxyéthyle et $R_4$ un groupement carboxyméthyle ; et

$$R_2\text{-CONHCH}_2\text{CH}_2\text{-N(B)(C)} \qquad (3)$$

dans laquelle :
B représente $-CH_2CH_2OX'$, C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$- CHOH - $SO_3H$
$R_2$, désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

A titre d'exemple on peut citer le cocoamphodiacetate commercialisé sous la dénomination commerciale MIRANOL C2M concentré NP par la Société RHODIA CHIMIE.

Tensioactifs cationiques :

Les tensioactifs cationiques peuvent être choisis parmi :

A) les sels d'ammonium quaternaires de la formule générale (XII) suivante :

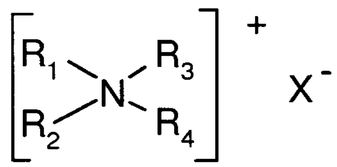

(XII)

dans laquelle $X^-$ est un anion choisi dans le groupe des halogénures (chlorure, bromure ou iodure) ou alkyl($C_2$-$C_6$) sulfates plus particulièrement méthylsulfate, des phosphates, des alkyl-ou-alkylarylsulfonates, des anions dérivés d'acide organique tel que l'acétate ou le lactate.
, et

i) les radicaux R1 à R3, qui peuvent être identiques ou différents, représentent un radical aliphatique, linéaire ou ramifié, comportant de 1 à 4 atomes de carbone,
ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
R4 désigne un radical alkyle, linéaire ou ramifié, comportant de 16 à 30 atomes de carbone.
De préférence le tensioactif cationique est un sel (par exemple chlorure) de cétyl triméthyl ammonium.
ii) les radicaux R1 et R2, qui peuvent être identiques ou différents, représentent un radical aliphatique, linéaire ou ramifié, comportant de 1 à 4 atomes de carbone,
ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
R3 et R4, identiques ou différents, désignent un radical alkyle, linéaire ou ramifié, comportant de 12 à 30 atomes de carbone, ledit radical comprenant au moins une fonction ester ou amide.
R3 et R4 sont notamment choisis parmi les radicaux alkyl($C_{12}$-$C_{22}$)amido alkyle($C_2$-$C_6$), alkyl($C_{12}$-$C_{22}$)acétate ;
De préférence le tensioactif cationique est un sel (par exemple chlorure) de stéaramidopropyl diméthyl (myristylacétate) ammonium.

B) - les sels d'ammonium quaternaire de l'imidazolinium, comme par exemple celui de formule (XIII) suivante :

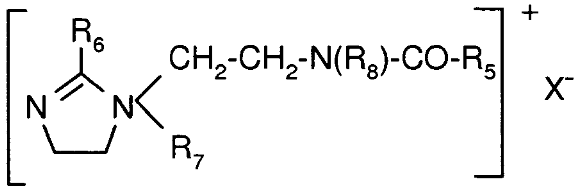

(XIII)

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical alkyle en $C_1$-$C_4$ , $R_8$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,

C) - les sels de diammonium quaternaire de formule (XIV) :

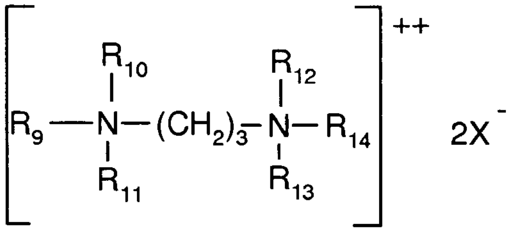

(XIV)

dans laquelle $R_9$ désigne un radical aliphatique comportant environ de 16 à 30 atomes de carbone, Rio, $R_{11}$, $R_{12}$, $R_{13}$ et $R_{14}$ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

D) - les sels d'ammonium quaternaire contenant au moins une fonction ester de formule (XV) suivante :

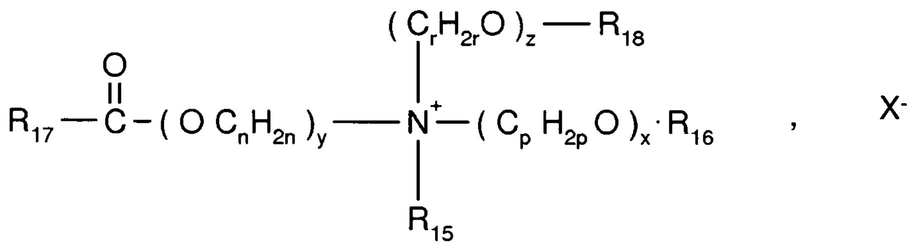

(XV)

dans laquelle :

- $R_{15}$ est choisi parmi les radicaux alkyles en $C_1$-$C_6$ et les radicaux hydroxyalkyles ou dihydroxyalkyles en $C_1$-$C_6$ ;
- $R_{16}$ est choisi parmi :

- le radical

- le radical 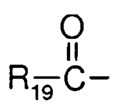

- les radicaux $R_{20}$ hydrocarbonés en $C_1$-$C_{22}$ linéaires ou ramifiés, saturés ou insaturés,
- l'atome d'hydrogène,
- $R_{18}$ est choisi parmi :

- le radical

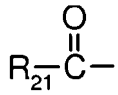

- les radicaux $R_{22}$ hydrocarbonés en $C_1$-$C_6$ linéaires ou ramifiés, saturés ou i nsatu rés,
- l'atome d'hydrogène,
- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;

sous réserve que la somme x + y + z vaut de 1 à 15 , que lorsque x vaut 0 alors $R_{16}$ désigne $R_{20}$ et que lorsque z vaut 0 alors $R_{18}$ désigne $R_{22}$.
On utilise plus particulièrement les sels d'ammonium de formule (XV) dans laquelle :

- $R_{15}$ désigne un radical méthyle ou éthyle,
- x et y sont égaux à 1 ;
- z est égal à 0 ou 1 ;
- n, p et r sont égaux à 2 ;
- $R_{16}$ est choisi parmi :

- le radical

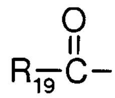

- les radicaux méthyle, éthyle ou hydrocarbonés en $C_{14}$-$C_{22}$
- l'atome d'hydrogène ;
- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- $R_{18}$ est choisi parmi :

- le radical

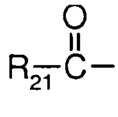

- l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société DEGUSSA-WITCO.

Parmi les sels d'ammonium quaternaire on préfère le chlorure de cétyltriméthylammonium, ou encore, le chlorure palmitamidopropyl triméthyl ammonium commercialisé sous la dénomination VARISOFT PA TC par la société DEGUSSA.

On utilise de préférence comme agent tensioactif anionique les alkyl($C_{12}$-$C_{14}$) sulfates de sodium, de triéthanolamine ou d'ammonium, les alkyl ($C_{12}$-$C_{14}$)éthersulfates de sodium, de triéthanolamine ou d'ammonium oxyéthylénés à 2,2 moles d'oxyde d'éthylène, le cocoyl iséthionate de sodium et l'alphaoléfine($C_{14}$-$C_{16}$) sulfonate de sodium et leurs mélanges avec :

- soit un agent tensioactif amphotère tel que les dérivés d'amine dénommés disodiumcocoamphodiacetate ou sodiumcocoamphopropionate commercialisés notamment par la société RHODIA CHIMIE sous la dénomination commerciale "MIRANOL® C2M CONCNP" en solution aqueuse à 38 % de matière active ou sous la dénomination MIRANOL® C32;
- soit un agent tensioactif amphotère tel que les alkylbétaïnes en particulier la cocobétaïne commercialisée sous la dénomination "DEHYTON® AB 30" en solution aqueuse à 32 % de MA par la société COGNIS ou tel que les alkyl ($C_8$-$C_{20}$) amidoalkyl ($C_1$-$C_6$) bétaïnes en particulier la TEGOBETAINE® F 50 commercialisée par la société DEGUSSA.

Selon une variante préférée de l'invention, les compositions cosmétiques peuvent également contenir des agents de conditionnement des matières kératiniques.

Ces compositions, lorsqu'elles sont appliquées sur les cheveux, possèdent de bonnes propriétés de conditionnement des cheveux, c'est-à-dire que les cheveux traités sont lisses, se démêlent facilement, sont doux au toucher. Les cheveux ont un aspect naturel et non chargé.

Lorsque la composition contient au moins un agent de conditionnement, ils sont généralement choisis parmi les huiles de synthèse telles que les poly-$\alpha$-oléfines, les huiles fluorées, les cires fluorées, les gommes fluorées, les esters gras d'acides carboxyliques différents de ceux de l'invention (ester liquide d'acide carboxylique ayant de 4 à 6 atomes de carbone et d'alcool ayant de 12 à 26 atomes de carbone), les polymères cationiques, les silicones, les huiles minérales, végétales ou animales, les céramides, les pseudocéramides et leurs mélanges.

Les polyoléfines sont de préférence des poly-$\alpha$-oléfines et en particulier :

- de type polybutène, hydrogéné ou non, et de préférence polyisobutène, hydrogéné ou non.
  On utilise de préférence les oligomères d'isobutylène de poids moléculaire inférieur à 1000 et leurs mélanges avec des polyisobutylènes de poids moléculaire supérieur à 1000 et de préférence allant de 1000 à 15000.
  A titre d'exemples de poly-$\alpha$-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.
  De tels produits sont vendus par exemple sous les dénominations ETHYLFLO par la société ETHYL CORP., et d'ARLAMOL PAO par la société ICI.

Les huiles minérales pouvant être utilisées dans les compositions de l'invention sont choisies préférentiellement dans le groupe formé par :

- les hydrocarbures, tels que l'hexadécane et l'huile de paraffine ;
  Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société AMERCHOL, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société NALCO, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL, les homopolymères et les copolymères éventuellement réticulés de sel de (méth)acryloyloxyéthyltriméthylammonium, vendus par la société CIBA en solution à 50% dans de l'huile minérale sous les dénominations

commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium). On peut également utiliser les polymères qui sont constitués de motifs récurrents répondant à la formule :

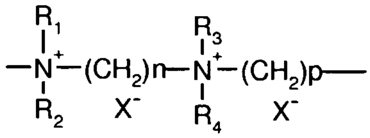 (a)

dans laquelle $R_1$, $R_2$, $R_3$ et $R_4$, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.

Les silicones utilisables conformément à l'invention sont en particulier des polyorganosiloxanes insolubles dans la composition et peuvent se présenter sous forme d'huiles, de cires, de résines ou de gommes.

Les silicones insolubles dans l'eau sont insolubles dans l'eau à une concentration supérieure ou égale à 0,1% en poids dans l'eau à 25°C, c'est à dire qu'elles ne forment pas une solution isotrope transparente.

La viscosité des silicones est par exemple mesurée à 25°C selon la norme ASTM 445 Appendice C.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition allant de 60° C à 260° C, et plus particulièrement encore parmi :

(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.

On peut également citer les cyclocopolymères du type diméthylsiloxanes/ méthylakylsiloxane, tel que la "SILICONE VOLATILE FZ 3109" commercialisée par la société UNION CARBIDE, de structure chimique :

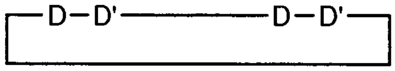

avec D : 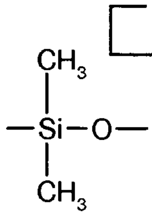 avec D' : 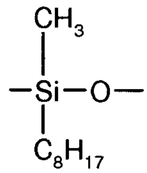

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;

(ii) les silicones volatiles linéaires ayant 2 à 9 atomes de silicium et possédant une viscosité inférieure ou égale à $5.10^{-6} m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de $5.10^{-6}$ à 2,5 $m^2/s$ à 25 °C et de préférence $1.10^{-5}$ à 1 $m^2/s$.

Parmi ces polyalkylsiloxanes, on peut citer à titre non limitatif les produits commerciaux suivants :

- les huiles de la série MIRASIL commercialisées par la société RHODIA CHIMIE, telles que par exemple l'huile

MIRASIL DM 500 000 ;

- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE.

Dans cette classe de polyalkylsiloxanes, on peut également citer les produits commercialisés sous les dénominations "ABIL WAX 9800 et 9801" par la société DEGUSSA qui sont des polyalkyl ($C_1$-$C_{20}$) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de $1.10^{-5}$ à $5.10^{-2}m^2/s$ à 25 °C.

Parmi ces polyalkylarylsiloxanes on peut citer à titre d'exemple les produits commercialisés sous les dénominations suivantes :

. les huiles MIRASIL DPDM de RHODIA CHIMIE ;
· les huiles des séries RHODORSIL 70 633 et 763 de RHODIA CHIMIE ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :

- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :

. les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6}m^2/s$. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :

$R_2SiO_{2/2}$, $R_3SiO_{1/2}$, $RSiO_{3/2}$ et $SiO_{4/2}$ dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en $C_1$-$C_4$, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC

et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl ($C_{12}$) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société DEGUSSA ;
- des groupements hydroxylés comme les polyorganosiloxanes à fonction hydroxyalkyle décrits dans la demande de brevet français FR-A-85 16334 .
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société DEGUSSA sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :

a) 50 à 90% en poids d'acrylate de tertiobutyle ;
b) 0 à 40% en poids d'acide acrylique ;
c) 5 à 40% en poids de macromère siliconé de formule :

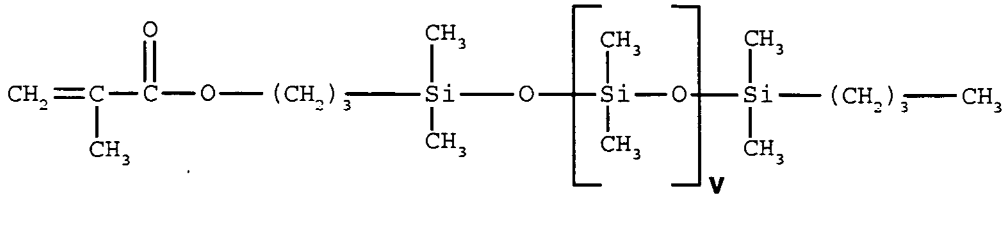

avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères

du type poly(méth)acrylate d'isobutyle.

Selon l'invention, toutes les silicones peuvent également être utilisées sous forme d'émulsions.

Les polyorganosiloxanes particulièrement préférés conformément à l'invention sont :

- les silicones non volatiles choisies dans la famille des polyalkylsiloxanes à groupements terminaux triméthylsilyle telles que les huiles ayant une viscosité comprise allant de 0,2 à 2,5 $m^2/s$ à 25° C telles que les huiles de la séries DC200 de DOW CORNING en particulier celle de viscosité 60 000 cSt, des séries MIRASIL DM et plus particulièrement l'huile MIRASIL DM 500 000 commercialisées par la société RHODIA CHIMIE ou l'huile de silicone AK 300.000 de la société WACKER, les polyalkylsiloxanes à groupements terminaux diméthylsilanol tels que les diméthiconol ou les polyalkylarylsiloxanes tels que l'huile MIRASIL DPDM commercialisée par la société RHODIA CHIMIE ;
- les polysiloxanes à groupements aminés tels que les amodiméthicones ou les triméthylsilylamodiméthicones ;

Selon la présente invention, les composés de type céramide sont notamment les céramides et/ou les glycocéramides et/ou les pseudocéramides et/ou les néocéramides, naturels ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131

Des composés de type céramides particulièrement préférés selon l'invention sont par exemple :

- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique
- le N-docosanoyl N-méthyl-D-glucamine

ou les mélanges de ces composés.

Selon l'invention, les agents conditionneurs différents des esters de l'invention peuvent représenter de 0,001 % à 10 % en poids, de préférence de 0,005 % à 5 % en poids, et encore plus préférentiellement de 0,01 % à 3 % en poids, du poids total de la composition finale.

Le milieu physiologiquement acceptable est de préférence constitué par de l'eau ou un mélange d'eau et de solvants cosmétiquement ou dermatologiquement acceptables tels que des monoalcools, des polyalcools, des éthers de polyol qui peuvent être utilisés seuls ou en mélange. L'eau représente de préférence de 30 à 98% en poids et de préférence de 50 à 98% en poids par rapport au poids total de la composition.

On peut citer plus particulièrement les monoalcools tels que l'éthanol, l'isopropanol, les polyalcools tels que le diéthylèneglycol, la glycérine, les éthers de polyol tels que les alkyléthers de propylèneglycol.

La composition de l'invention peut également contenir au moins un additif choisi parmi les séquestrants, les adoucissants, les modificateurs de mousse, les colorants, d'autres agents nacrants, les agents hydratants, les agents antipelliculaires ou anti-séborrhéiques, d'autres agents de mise en suspension, les hydroxyacides, les épaississants, les esters d'acides gras, les parfums, les conservateurs, les filtres solaires, les protéines, les vitamines et provitamines, les polymères, et tout autre additif classiquement utilisé dans le domaine cosmétique.

Ces additifs sont présents dans la composition selon l'invention dans des proportions pouvant aller de 0 à 40% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion épaissie ou non ou de mousse.

Les compositions conformes à l'invention peuvent être utilisées pour le traitement des matières kératiniques telles que les cheveux, la peau, les cils, les sourcils, les ongles, les lèvres, le cuir chevelu et plus particulièrement les cheveux.

Les compositions peuvent également être utilisées pour le lavage et le nettoyage des matières kératiniques telles que les cheveux et la peau.

Les compositions selon l'invention sont utilisées généralement comme produits notamment pour le lavage, le soin, le conditionnement, le maintien de la coiffure ou la mise en forme des matières kératiniques telles que les cheveux.

Les compositions de l'invention peuvent plus particulièrement se présenter sous forme de shampooing, d'après-shampooing à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage. De préférence, les compositions sont des compositions lavantes et moussantes pour les cheveux et/ou la peau.

En particulier, les compositions selon l'invention sont des compositions détergentes moussantes telles que des shampooings, des gels-douche et des bains moussants. Dans ce mode de réalisation de l'invention, les compositions comprennent au moins un tensioactif détergent.

Le ou les tensioactifs détergents peuvent être indifféremment choisis, seuls ou en mélanges, au sein des tensioactifs anioniques, amphotères, non ioniques, zwittérioniques et cationiques décrits ci-dessus et plus particulièrement parmi les tensioactifs anioniques et les mélanges de tensioactifs anioniques et de tensioactifs amphotères et/ou non ioniques.

La quantité minimale en tensioactif est celle juste suffisante pour conférer à la composition finale un pouvoir moussant et/ou détergent satisfaisant.

Ainsi, selon l'invention, le tensioactif détergent peut avantageusement représenter de 3 % à 30 % en poids, de préférence de 6 % à 25 % en poids, et encore plus préférentiellement de 8 % à 20 % en poids, du poids total de la composition finale.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.

Les modifications de la méthode sont les suivantes :

La mesure se fait à la température de 22°C avec de l'eau osmosée. La concentration de la solution est de 2g/l. La hauteur de la chute est de 1 m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Lorsque la composition se présente sous la forme d'un après-shampooing éventuellement à rincer, elle contient avantageusement un tensioactif cationique, sa concentration allant généralement de 0,1 à 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux. Les compositions sont de préférence liquides.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

L'invention a encore pour objet un procédé de traitement cosmétique des matières kératiniques telles que les cheveux consistant à appliquer sur ceux-ci une composition telle que définie précédemment puis à effectuer éventuellement un rinçage à l'eau, après un éventuel temps de pause.

L'invention va être maintenant plus complètement illustrée à l'aide des exemples suivants qui ne sauraient être considérés comme la limitant aux modes de réalisation décrits. Dans ce qui suit, MA signifie Matière Active.

## EXEMPLES

On a préparé deux shampooings conformes à l'invention B et C et un shampooing A (non conforme à l'invention):

| % MA | A (comparatif) | B | C |
|---|---|---|---|
| | | | |
| Eau qsp | 100 | 100 | 100 |
| Polyquaternium-10 (UCARE POLYMER JR400 de AMERCHOL) | 0,4 | 0,4 | 0,4 |
| Carbomer (CARBOPOL 980 de NOVEON) | 0,2 | 0,2 | 0,2 |
| Néopentanoate d'isostéaryle (CERAPHYL 375 de ISP) | | 0,5 | 0,25 |
| ß-Cyclodextrine (Cavamax W7 de WACKER) | 2 | 2 | 2 |
| Lauryléthersulfate de sodium à 2.2 moles d'oxyde d'éthylène | 14 | 14 | 14 |
| Cocamidopropyl bétaine | 2,4 | 2,4 | 2,4 |
| Dimethicone (MIRASIL DM 500.000 de RHODIA) | 1,5 | 1,5 | 1,5 |
| Vitamines : (Nicotinamide de DSM, d-Panthénol de BASF, dl-alpha tocophéryl acétate de DSM) | 0,3 | 0,3 | 0,3 |
| Conservateur | 0,45 | 0,45 | 0,45 |
| Parfum | qs | qs | qs |
| Hydroxyde de sodium / Acide Citrique qs | pH 6,8 - 7 | pH 6,8 - 7 | pH 6,8 - 7 |
| | | | |
| viscosité (temps d'écoulement en sec, C Ford 10) | 55 | 69 | 58 |

Les compositions B et C sont stables plus de 2 mois à température ambiante et à 45°C. Elle a un bel effet nacré et présente de bonnes propriétés cosmétiques. Les cheveux se démêlent facilement et sont lisses.

La composition A est instable (présence de 2 phases) au bout de 51 jours à 45°C.

On a préparé un shampooing conforme à l'invention

| % MA | |
|---|---|
| Polyquaternium-10 (UCARE POLYMER JR400 de AMERCHOL) | 1,2 |
| Carbomer (CARBOPOL 980 de NOVEON) | 0,2 |
| Néopentanoate de tridécyle (CERAPHYL 55 de ISP) | 0,5 |
| ß-Cyclodextrine (Cavamax W7 de WACKER) | 2 |
| Lauryléthersulfate de sodium à 2.2 moles d'oxyde d'éthylène | 11,2 |
| Cocamide MIPA (Empilan CIS de Huntsman) | 1 |
| Disodium Cocoamphodiacétate (MIRANOL C2M conc. de RHODIA) | 3 |
| Hydrolysat de protéine de blé quaternisée (HYDROTRITICUM WQ1) | 0,3 |
| Ester de glucose et de vitamine F | 0,01 |
| Ester de glycérol et de vitamine F | 0,1 |
| Glycérine | 1 |
| Conservateur | 1,2 |
| Parfum | qs |
| Hydroxyde de sodium / Acide Citrique qs | pH 5-5,5 |
| Eau qsp | 100 |

## Revendications

1. Composition cosmétique **caractérisée en ce qu'**elle comprend dans un milieu aqueux cosmétiquement acceptable, au moins un agent tensioactif anionique, au moins un ester liquide d'acide carboxylique ayant de 4 à 6 atomes de carbone et d'alcool ayant de 12 à 26 atomes de carbone, au moins une cyclodextrine ou l'un de ses dérivés et au moins un polymère épaississant acrylique.

2. Composition selon la revendication 1, **caractérisée par le fait que** les cyclodextrines ou l'un de leurs dérivés sont choisies parmi les α-cyclodextrines, les β-cyclodextrines ou les γ-cyclodextrines ou l'un de leurs dérivés.

3. Composition selon la revendication 2, **caractérisée par le fait que** les cyclodextrines sont choisies parmi les β-cyclodextrines ou les γ-cyclodextrines, de préférence parmi les β-cyclodextrines .

4. Composition selon l'une quelconque des revendications 1 à 3, **caractérisée en ce que** la cyclodextrine ou l'un de ses dérivés représente de 1 % à 10 % en poids, et encore plus préférentiellement de 1,5 % à 5 % en poids, par rapport au poids total de la composition finale.

5. Composition selon l'une quelconque des revendications 1 à 4, **caractérisée en ce que** le tensioactif et la cyclodextrine ou l'un de ses dérivés sont présents dans des concentrations efficaces pour former un complexe insoluble dans la composition, et/ou pour nacrer la composition.

6. Composition selon l'une quelconque des revendications 1 à 5, **caractérisée par le fait que** ledit ester présente la formule suivante :

$$R_1\ COOR_2 \qquad (I)$$

dans laquelle :

$R_1$ désigne un radical hydrocarboné, linéaire ou ramifié, éventuellement mono ou polyhydroxylé, ayant de 3 à 5 atomes de carbone,
$R_2$ désigne un radical hydrocarboné, linéaire ou ramifié, éventuellement mono ou polyhydroxylé, ayant de 12 à 26 atomes de carbone,

7. Composition selon la revendication 6, **caractérisée par le fait que** $R_1$ désigne un radical alkyle ramifié ayant de 3 à 5 atomes de carbone,
$R_2$ désigne un radical alkyle ayant 12 à 26 atomes de carbone, plus particulièrement ramifié.

8. Composition selon l'une quelconque des revendications 6 ou 7, **caractérisée par le fait que** $R_1$ désigne un radical tertio-butyle,

9. Composition selon l'une quelconque des revendications 6 à 8, **caractérisée par le fait que** $R_2$ est choisi parmi les radicaux tridécyle, isocétyle, isostéaryle, octyldodécyle et isoarachidyle.

10. Composition selon l'une quelconque des revendications 1 à 9, **caractérisée par le fait que** ledit ester est présent dans des concentrations comprises entre 0,1 et 20 %, et de préférence entre 0,2 et 10 % en poids par rapport au poids total de la composition.

11. Composition selon l'une quelconque des revendications 1 à 10, **caractérisée par le fait que** ledit polymère épaississant acrylique est choisi parmi :

(a) les épaississants associatifs acryliques;
(b) les homopolymères d'acide acrylique réticulés ;
(c) les copolymères réticulés d'acide (méth)acrylique et d'acrylate d'alkyle($C_1$-$C_6$),
(d) les homopolymères et copolymères non-ioniques contenant des monomères à insaturation éthylénique de type ester et/ou amide ;
(e) les homopolymères d'acrylate d'ammonium ou les copolymères d'acrylate d'ammonium et d'acrylamide ;
(f) les homopolymères et copolymères d'acides (méth)acrylamido alkyl(C1-C4) sulfoniques
(g) les homopolymères et copolymères de méthacryoyl alkyl(C1-C4) trialkyl(C1-C4) ammonium réticulés.

**12.** Composition selon l'une quelconque des revendications 1 à 11, **caractérisée par le fait que** ledit polymère épaississant acrylique est choisi parmi :

(a) les épaississants associatifs acryliques;
(b) les homopolymères d'acide acrylique réticulés ;
(c) les copolymères réticulés d'acide (méth)acrylique et d'acrylate d'alkyle($C_1$-$C_6$)

**13.** Composition selon la revendication 12, **caractérisée par le fait que** ledit polymère épaississant associatif acrylique est choisi parmi les polymères amphiphiles anioniques acryliques comportant au moins un motif hydrophile de formule (IV), et au moins un motif hydrophobe de type ester d'alkyl ($C_{10}$-$C_{30}$) d'acide carboxylique insaturé de formule (V) suivante :

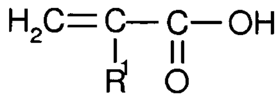

(IV)

formule dans laquelle, $R^1$ désigne H ou $CH_3$ ou $C_2H_5$, c'est-à-dire des motifs acide acrylique, acide méthacrylique ou acide éthacrylique et dont le motif hydrophobe de type ester d'alkyl ($C_{10}$-$C_{30}$) d'acide carboxylique insaturé correspond au monomère de formule (V) suivante :

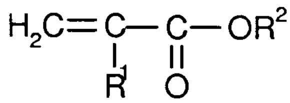

(V)

formule dans laquelle, $R^1$ désigne H ou $CH_3$ ou $C_2H_5$ (c'est-à-dire des motifs acrylates, méthacrylates ou éthacrylates) et de préférence H (motifs acrylates) ou $CH_3$ (motifs méthacrylates), $R^2$ désignant un radical alkyle en $C_{10}$-$C_{30}$, et de préférence en $C_{12}$-$C_{22}$.

**14.** Composition selon l'une quelconque des revendications 1 à 13, **caractérisée en ce que** ledit polymère épaississant acrylique représente de 0,001 % à 20 % en poids, de préférence de 0,01 % à 10% en poids et plus particulièrement de 0,1 à 3% en poids par rapport au poids total de la composition finale.

**15.** Composition selon l'une quelconque des revendications 1 à 14, **caractérisée en ce que** ledit tensioactif anionique est choisi parmi les sels (en particulier sels alcalins, notamment de sodium, sels d'ammonium, sels d'amines, sels d'aminoalcools ou sels de magnésium) des composés suivants : les alkyl sulfates, les alkyl éther sulfates, alkyl amido éther sulfates, alkyl aryl polyéther sulfates, monoglycérides sulfates ; les alkyl sulfonates, alkylphosphates, alkyl amido sulfonates, alkyl aryl sulfonates, $\alpha$-oléfine-sulfonates, paraffine-sulfonates ; les alkyl sulfo succinates, les alkyl éther sulfosuccinates, les alkyl amide sulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle, les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone, les acides d'alkyl D galactoside uroniques et leurs sels ainsi que les acides alkyl ($C_8$-$C_{24}$) éther carboxyliques polyoxyalkylénés, les acides alkyl($C_8$-$C_{24}$)aryl éther carboxyliques polyoxyalkylénés, les acides alkylamido($C_8$-$C_{24}$) éther carboxyliques polyoxyalkylénés et leurs sels, en particulier ceux comportant de 2 à 50 groupements oxyde d'éthylène, et leurs mélanges.

**16.** Composition selon l'une quelconque des revendications 1 à 15, **caractérisée en ce que** le tensioactif anionique est présent en une quantité comprise allant de 4% à 35% et encore plus préférentiellement de 8 % à 30%, par rapport au poids total de la composition

**17.** Composition selon l'une quelconque des revendications précédentes, **caractérisée en ce qu'**elle comprend en outre au moins un agent de conditionnement.

**18.** Composition selon la revendication précédente, **caractérisée en ce que** l'agent de conditionnement est choisi parmi les poly-α-oléfines, les huiles fluorées, les cires fluorées, les gommes fluorées, les esters d'acides carboxyliques différents des esters liquide d'acide carboxylique ayant de 4 à 6 atomes de carbone et d'alcool ayant de 12 à 26 atomes de carbone, les silicones, les polymères cationiques, les huiles minérales, végétales ou animales, les céramides, les pseudocéramides, et leurs mélanges.

**19.** Composition selon l'une des revendications 17 ou 18, **caractérisée en ce** les agents conditionneurs représentent de 0,001 % à 10 % en poids, de préférence de 0,005 % à 5 % en poids, et encore plus préférentiellement de 0,01 % à 3 % en poids, du poids total de la composition.

**20.** Composition selon l'une quelconque des revendications précédentes, **caractérisée par le fait qu'**elle se présente sous forme de gel, de lait, de crème, de lotion ou de mousse.

**21.** Composition selon l'une quelconque des revendications précédentes, **caractérisée par le fait qu'**elle constitue une composition détergente moussante telles que des shampooings, des gels-douche et des bains moussants.

**22.** Composition selon l'une quelconque des revendications précédentes, **caractérisée par le fait qu'**elle constitue une composition d'après-shampooing à rincer ou non, des compositions pour permanente, défrisage, coloration ou décoloration, des compositions à appliquer avant ou après une coloration, une décoloration, une permanente ou un défrisage ou encore entre les deux étapes d'une permanente ou d'un défrisage.

**23.** Procédé de traitement cosmétique des matières kératiniques en particulier des cheveux, **caractérisé par le fait qu'**il consiste à appliquer sur lesdites matières une composition telle que définie selon l'une quelconque des revendications 1 à 22 puis à effectuer éventuellement un rinçage à l'eau.

**24.** Procédé pour améliorer la stabilité au stockage d'une composition cosmétique comprenant au moins une cyclodextrine et au moins un tensioactif consistant à associer dans ladite composition un polymère acrylique épaississant et un ester liquide d'acide carboxylique ayant de 4 à 6 atomes de carbone et d'alcool ayant de 12 à 26 atomes de carbone.

## Claims

**1.** Cosmetic composition, **characterized in that** it comprises, in a cosmetically acceptable aqueous medium, at least one anionic surfactant, at least one liquid ester of a carboxylic acid containing from 4 to 6 carbon atoms and of an alcohol containing from 12 to 26 carbon atoms, at least one cyclodextrin or a derivative thereof, and at least one acrylic thickening polymer.

**2.** Composition according to Claim 1, **characterized in that** the cyclodextrins or derivative thereof are chosen from α-cyclodextrins, β-cyclodextrins and γ-cyclodextrins, or a derivative thereof.

**3.** Composition according to Claim 2, **characterized in that** the cyclodextrins are chosen from β-cyclodextrins and γ-cyclodextrins, preferably from β-cyclodextrins.

**4.** Composition according to any one of Claims 1 to 3, **characterized in that** the cyclodextrin or derivative thereof represents from 1% to 10% by weight and even more preferably from 1.5% to 5% by weight relative to the total weight of the final composition.

**5.** Composition according to any one of Claims 1 to 4, **characterized in that** the surfactant and the cyclodextrin or derivative thereof are present in concentrations that are effective to form an insoluble complex in the composition, and/or to make the composition nacreous.

**6.** Composition according to any one of Claims 1 to 5, **characterized in that** the said ester has the following formula:

$$R_1COOR_2 \qquad (I)$$

in which:

$R_1$ denotes a linear or branched, optionally mono- or polyhydroxylated hydrocarbon-based radical containing from 3 to 5 carbon atoms,
$R_2$ denotes a linear or branched, optionally mono- or polyhydroxylated hydrocarbon-based radical containing from 12 to 26 carbon atoms.

**7.** Composition according to Claim 6, **characterized in that** $R_1$ denotes a branched alkyl radical containing from 3 to 5 carbon atoms,
$R_2$ denotes an alkyl radical containing from 12 to 26 carbon atoms, which is more particularly branched.

**8.** Composition according to either of Claims 6 and 7, **characterized in that** $R_1$ denotes a tert-butyl radical.

**9.** Composition according to any one of Claims 6 to 8, **characterized in that** $R_2$ is chosen from tridecyl, isocetyl, isostearyl, octyldodecyl and isoarachidyl radicals.

**10.** Composition according to any one of Claims 1 to 9, **characterized in that** the said ester is present in concentrations of between 0.1% and 20% and preferably between 0.2% and 10% by weight relative to the total weight of the composition.

**11.** Composition according to any one of Claims 1 to 10, **characterized in that** the said acrylic thickening polymer is chosen from:

(a) acrylic associative thickeners;
(b) crosslinked acrylic acid homopolymers;
(c) crosslinked copolymers of (meth)acrylic acid and of a $(C_1-C_6)$alkyl acrylate;
(d) nonionic homopolymers and copolymers containing ethylenically unsaturated monomers of ester and/or amide type;
(e) ammonium acrylate homopolymers or copolymers of ammonium acrylate and of acrylamide;
(f) (meth)acrylamido$(C_1-C_4)$alkylsulfonic acid homopolymers and copolymers;
(g) crosslinked methacryloyl$(C_1-C_4)$alkyltri$(C_1-C_4)$alkylammonium homopolymers and copolymers.

**12.** Composition according to any one of Claims 1 to 11, **characterized in that** the said acrylic thickening polymer is chosen from:

(a) acrylic associative thickeners;
(b) crosslinked acrylic acid homopolymers;
(c) crosslinked copolymers of (meth)acrylic acid and of a $(C_1-C_6)$alkyl acrylate.

**13.** Composition according to Claim 12, **characterized in that** the said acrylic associative thickening polymer is chosen from acrylic anionic amphiphilic polymers comprising at least one hydrophilic unit of formula (IV) and at least one hydrophobic unit of the type such as a $(C_{10}-C_{30})$alkyl ester of an unsaturated carboxylic acid of formula (V) below:

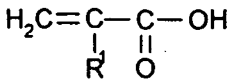
(IV)

in which formula $R^1$ denotes H or $CH_3$ or $C_2H_5$, i.e. acrylic acid, methacrylic acid or ethacrylic acid units, and in which the hydrophobic unit of the type such as a $(C_{10}-C_{30})$ alkyl ester of an unsaturated carboxylic acid corresponds to the monomer of formula (V) below:

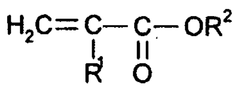
(V)

in which formula $R^1$ denotes H or $CH_3$ or $C_2H_5$ (i.e. acrylate, methacrylate or ethacrylate units) and preferably H

(acrylate units) or $CH_3$ (methacrylate units), $R^2$ denoting a $C_{10}$-$C_{30}$ and preferably $C_{12}$-$C_{22}$ alkyl radical.

**14.** Composition according to any one of Claims 1 to 13, **characterized in that** the said acrylic thickening polymer represents from 0.001% to 20% by weight, preferably from 0.01% to 10% by weight and more particularly from 0.1% to 3% by weight relative to the total weight of the final composition.

**15.** Composition according to any one of Claims 1 to 14, **characterized in that** the said anionic surfactant is chosen from salts (in particular alkaline salts, especially sodium salts, ammonium salts, amine salts, amino alcohol salts or magnesium salts) of the following compounds: alkyl sulfates, alkyl ether sulfates, alkylamido ether sulfates, alkylarylpolyether sulfates, monoglyceride sulfates; alkyl sulfonates, alkyl phosphates, alkylamide sulfonates, alkylaryl sulfonates, $\alpha$-olefin sulfonates, paraffin sulfonates; alkyl sulfosuccinates, alkyl ether sulfosuccinates, alkylamide sulfosuccinates; alkyl sulfosuccinamates; alkyl sulfoacetates; alkyl ether phosphates; acyl sarcosinates; acyl isethionates and N-acyltaurates, the alkyl or acyl radical of all of these various compounds preferably containing from 8 to 24 carbon atoms, and the aryl radical preferably denoting a phenyl or benzyl group; fatty acid salts such as the salts of oleic, ricinoleic, palmitic and stearic acids, coconut oil acid or hydrogenated coconut oil acid; acyl lactylates in which the acyl radical contains 8 to 20 carbon atoms; alkyl-D-galactosiduronic acids and their salts, as well as polyoxyalkylenated ($C_8$-$C_{24}$) alkyl ether carboxylic acids, polyoxyalkylenated ($C_8$-$C_{24}$) alkylaryl ether carboxylic acids, polyoxyalkylenated ($C_8$-$C_{24}$) alkylamido ether carboxylic acids and their salts, in particular those containing from 2 to 50 ethylene oxide groups, and mixtures thereof.

**16.** Composition according to any one of Claims 1 to 15, **characterized in that** the anionic surfactant is present in an amount ranging from 4% to 35% and even more preferentially from 8% to 30% relative to the total weight of the composition.

**17.** Composition according to any one of the preceding claims, **characterized in that** it also comprises at least one conditioning agent.

**18.** Composition according to the preceding claim, **characterized in that** the conditioning agent is chosen from poly-$\alpha$-olefins, fluoro oils, fluoro waxes, fluoro gums, carboxylic acid esters other than the liquid esters of a carboxylic acid containing from 4 to 6 carbon atoms and of an alcohol containing from 12 to 26 carbon atoms, silicones, cationic polymers, mineral, plant or animal oils, ceramides and pseudoceramides, and mixtures thereof.

**19.** Composition according to either of Claims 17 and 18, **characterized in that** the conditioning agents represent from 0.001% to 10% by weight, preferably from 0.005% to 5% by weight and even more preferentially from 0.01% to 3% by weight relative to the total weight of the composition.

**20.** Composition according to any one of the preceding claims, **characterized in that** it is in the form of a gel, a milk, a cream, a lotion or a mousse.

**21.** Composition according to any one of the preceding claims, **characterized in that** it constitutes a foaming detergent composition, such as shampoos, shower gels and bubble baths.

**22.** Composition according to any one of the preceding claims, **characterized in that** it constitutes a rinse-out or leave-in hair-conditioning composition, permanent-waving, relaxing, dyeing or bleaching compositions, or compositions to be applied before or after dyeing, bleaching, permanent-waving or relaxing the hair, or alternatively between the two steps of a permanent-waving or hair-relaxing operation.

**23.** Cosmetic process for treating keratin materials, in particular the hair, **characterized in that** it consists in applying to the said materials a composition as defined according to any one of Claims 1 to 22, and then optionally rinsing with water.

**24.** Process for improving the stability on storage of a cosmetic composition comprising at least one cyclodextrin and at least one surfactant, which consists in combining in the said composition a thickening acrylic polymer and a liquid ester of a carboxylic acid containing from 4 to 6 carbon atoms and of an alcohol containing from 12 to 26 carbon atoms.

**Patentansprüche**

1. Kosmetische Zusammensetzung, **dadurch gekennzeichnet, dass** sie in einem kosmetisch akzeptablen wässerigen Medium enthält:

   mindestens ein anionisches Tensid,
   mindestens einen flüssigen Ester einer Carbonsäure mit 4 bis 6 Kohlenstoffatomen mit einem Alkohol mit 12 bis 26 Kohlenstoffatomen,
   mindestens ein Cyclodextrin oder eines seiner Derivate
   und
   mindestens ein verdickendes Acrylpolymer.

2. Zusammensetzung nach Anspruch 1, **dadurch gekennzeichnet, dass** die Cyclodextrine oder eines ihrer Derivate ausgewählt sind unter den α-Cyclodextrinen, den β-Cyclodextrinen oder den γ-Cyclodextrinen oder einem ihrer Derivate.

3. Zusammensetzung nach Anspruch 2, **dadurch gekennzeichnet, dass** die Cyclodextrine unter den β-Cyclodextrinen oder den γ-Cyclodextrinen ausgewählt sind, vorzugsweise unter den β-Cyclodextrinen.

4. Zusammensetzung nach einem der Ansprüche 1 bis 3, **dadurch gekennzeichnet, dass** das Cyclodextrin oder eines seiner Derivate in einem Mengenanteil von 1 bis 10 Gew.-%, noch bevorzugter in einem Mengenanteil von 1,5 bis 5 Gew.-% vorliegt, bezogen auf das Gesamtgewicht der fertigen Zusammensetzung.

5. Zusammensetzung nach einem der Ansprüche 1 bis 4, **dadurch gekennzeichnet, dass** das Tensid und das Cyclodextrin oder eines seiner Derivate in wirksamen Konzentrationen vorliegen, um einen in der Zusammensetzung unlöslichen Komplex zu bilden und/oder der Zusammensetzung einen Perlglanz zu verleihen.

6. Zusammensetzung nach einem der Ansprüche 1 bis 5, **dadurch gekennzeichnet, dass** der Ester die nachstehende Formel aufweist:

   $$R_1COOR_2 \qquad (I),$$

   in der bedeuten:

   $R_1$ eine geradkettige oder verzweigte und gegebenenfalls mono- oder polyhydroxylierte Kohlenwasserstoffgruppe mit 3 bis 5 Kohlenstoffatomen
   und
   $R_2$ eine geradkettige oder verzweigte und gegebenenfalls mono- oder polyhydroxylierte Kohlenwasserstoffgruppe mit 12 bis 26 Kohlenstoffatomen.

7. Zusammensetzung nach Anspruch 6, **dadurch gekennzeichnet, dass** $R_1$ eine verzweigte Alkylgruppe mit 3 bis 5 Kohlenstoffatomen und $R_2$ eine Alkylgruppe mit 12 bis 26 Kohlenstoffatomen, insbesondere eine verzweigte Alkylgruppe, bedeuten.

8. Zusammensetzung nach Anspruch 6 oder 7, **dadurch gekennzeichnet, dass** $R_1$ eine tert.-Butylgruppe bedeutet.

9. Zusammensetzung nach einem der Ansprüche 6 bis 8, **dadurch gekennzeichnet, dass** $R_2$ unter den Gruppen Tridecyl, Isocetyl, Isostearyl, Octyldodecyl und Isoarachidyl ausgewählt ist.

10. Zusammensetzung nach einem der Ansprüche 1 bis 9, **dadurch gekennzeichnet, dass** der Ester in Konzentrationen im Bereich von 0,1 bis 20 Gew.-% und bevorzugt im Bereich von 0,2 bis 10 Gew.-% vorliegt, bezogen auf das Gesamtgewicht der Zusammensetzung.

11. Zusammensetzung nach einem der Ansprüche 1 bis 10, **dadurch gekennzeichnet, dass** das verdickende Acrylpolymer ausgewählt ist unter:

    (a) assoziativen Acryl-Verdickungsmitteln;
    (b) vernetzten Homopolymeren von Acrylsäure;

(c) vernetzten Copolymeren von (Meth)acrylsäure und $C_1$-$C_6$-Alkylacrylaten;
(d) nichtionischen Homopolymeren und Copolymeren, die ethylenisch ungesättigte Monomere vom Ester- und/oder Amidtyp enthalten;
(e) Homopolymeren von Ammoniumacrylat oder Copolymeren von Ammoniumacrylat und Acrylamid;
(f) Homopolymeren und Copolymeren von (Meth)acrylamido-$C_1$-$C_4$-alkylsulfonsäuren;
(g) vernetzten Homopolymeren und Copolymeren von Methacryloyl-$C_1$-$C_4$-alkyl-tri-$C_1$-$C_4$-alkylammoniumsalzen.

**12.** Zusammensetzung nach einem der Ansprüche 1 bis 11, **dadurch gekennzeichnet, dass** das verdickende Acrylpolymer ausgewählt ist unter

(a) assoziativen Acryl-Verdickungsmitteln;
(b) vernetzten Homopolymeren von Acrylsäure;
(c) vernetzten Copolymeren von (Meth)acrylsäure und $C_1$-$C_6$-Alkylacrylaten.

**13.** Zusammensetzung nach Anspruch 12, **dadurch gekennzeichnet, dass** das assoziative verdickende Acrylpolymer ausgewählt ist unter amphiphilen anionischen Acrylpolymeren, die mindestens eine hydrophile Einheit der Formel (IV) und mindestens eine hydrophobe Einheit vom Typ eines $C_{10}$-$C_{30}$-Alkylesters einer ungesättigten Carbonsäure der nachstehenden Formel (V) aufweisen,

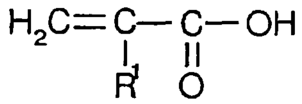
(IV),

in der bedeuten:

$R_1$ H oder $CH_3$ oder $C_2H_5$, das heißt, Einheiten von Acrylsäure, Methacrylsäure oder Ethacrylsäure,
und wobei die hydrophobe Einheit vom Typ eines $C_{10}$-$C_{30}$-Alkylesters einer ungesättigten Carbonsäure dem Monomer der nachstehenden Formel (V) entspricht,

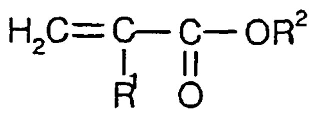
(V),

in der bedeuten:

$R_1$ H oder $CH_3$ oder $C_2H_5$ (d.h., Acrylat-, Methacrylat- oder Ethacrylat-Einheiten) und bevorzugt H (Acryleinheiten) oder $CH_3$ (Methacrylat-Einheiten), und
$R_2$ eine $C_{10}$-$C_{30}$-Alkylgruppe und vorzugsweise eine $C_{12}$-$C_{22}$-Alkylgruppe.

**14.** Zusammensetzung nach einem der Ansprüche 1 bis 13, **dadurch gekennzeichnet, dass** das verdickende Acrylpolymer in einem Mengenanteil von 0,001 bis 20 Gew.-%, bevorzugt in einem Mengenanteil von 0,01 bis 10 Gew.-% und noch bevorzugter in einem Mengenanteil von 0,1 bis 3 Gew.-% vorliegt, bezogen auf das Gesamtgewicht der fertigen Zusammensetzung.

**15.** Zusammensetzung nach einem der Ansprüche 1 bis 14, **dadurch gekennzeichnet, dass** das anionische Tensid ausgewählt ist unter den Salzen (insbesondere den Alkalisalzen, besonders den Natriumsalzen, den Ammoniumsalzen, den Aminsalzen, den Salzen von Aminoalkoholen oder den Magnesiumsalzen) von folgenden Verbindungen: den Alkylsulfaten, den Alkylethersulfaten, Alkylamidoethersulfaten, Alkylarylpolyethersulfaten, Monoglyceridsulfaten; den Alkylsulfonaten, Alkylphosphaten, Alkylamidosulfonaten, Alkylarylsulfonaten, $\alpha$-Olefinsulfonaten, Paraffinsulfonaten; den Alkylsulfosuccinaten, den Alkylethersulfosuccinaten, den Alkylamidsulfosuccinaten; den Alkylsulfosuccinamaten, den Alkylsulfoacetaten; den Alkyletherphosphaten; den Alkylsarcosinaten; den Acylisethionaten

und den N-Acyltauraten, wobei die Alkylgruppe oder Acylgruppe aller dieser verschiedenen Verbindungen vorzugsweise 8 bis 24 Kohlenstoffatome aufweist und die Arylgruppe bevorzugt eine Phenylgruppe oder Benzylgruppe darstellt, den Salzen von Fettsäuren, wie den Salzen von Ölsäure, Ricinolsäure, Palmitinsäure, Stearinsäure, den Copraölsäuren oder den Säuren von hydriertem Copraöl; den Acyllactylaten, deren Acylgruppe 8 bis 20 Kohlenstoffatome aufweist, den Alkyl-D-Galactosiduronsäuren und ihren Salzen sowie den polyoxyalkylenierten $C_8$-$C_{24}$-Alkylethercarbonsäuren, den polyoxyalkylenierten $C_8$-$C_{24}$-Alkylarylethercarbonsäuren, den polyoxyalkylenierten $C_8$-$C_{24}$-Alkylamidoethercarbonsäuren und ihren Salzen, insbesondere den Verbindungen mit 2 bis 50 Ethylenoxidgruppen, sowie den Gemischen dieser Verbindungen.

**16.** Zusammensetzung nach einem der Ansprüche 1 bis 15, **dadurch gekennzeichnet, dass** das anionische Tensid in einem Mengenanteil von 4 bis 35 % und noch bevorzugter in einem Mengenanteil von 8 bis 30 % vorliegt, bezogen auf das Gesamtgewicht der Zusammensetzung.

**17.** Zusammensetzung nach einem der vorhergehenden Ansprüche, **dadurch gekennzeichnet, dass** sie ferner mindestens ein Konditioniermittel enthält.

**18.** Zusammensetzung nach dem vorhergehenden Anspruch, **dadurch gekennzeichnet, dass** das Konditioniermittel ausgewählt ist unter Poly-$\alpha$-olefinen, fluorierten Ölen, fluorierten Wachsen, fluorierten Kautschuken, Carbonsäureestern, die von Estern einer Carbonsäure mit 4 bis 6 Kohlenstoffatomen mit einem Alkohol mit 12 bis 26 Kohlenstoffatomen verschieden sind, Siliconen, kationischen Polymeren, Mineralölen, Pflanzenölen oder tierischen Ölen, Ceramiden und Pseudoceramiden sowie Gemischen dieser Verbindungen.

**19.** Zusammensetzung nach Anspruch 17 oder 18, **dadurch gekennzeichnet, dass** die Konditioniermittel in einem Mengenanteil von 0,001 bis 10 Gew.-%, bevorzugt in einem Mengenanteil von 0,005 bis 5 Gew.-% und noch bevorzugter in einem Mengenanteil von 0,01 bis 3 Gew.-% vorliegen, bezogen auf das Gesamtgewicht der Zusammensetzung.

**20.** Zusammensetzung nach einem der vorhergehenden Ansprüche, **dadurch gekennzeichnet, dass** sie in Form eines Gels, einer Milch, einer Creme, einer Lotion oder eines Schaums vorliegt.

**21.** Zusammensetzung nach einem der vorhergehenden Ansprüche, **dadurch gekennzeichnet, dass** sie eine schäumende Reinigungszusammensetzung darstellt, wie Shampoos, Duschgels und Schaumbäder.

**22.** Zusammensetzung nach einem der vorhergehenden Ansprüche, **dadurch gekennzeichnet, dass** sie eine Apres-ShampooZusammensetzung, die ausgespült oder nicht ausgespült wird, eine Zusammensetzung für Dauerwellen, eine Zusammensetzung zum Entkrausen, eine Zusammensetzung zum Färben oder zum Entfärben, eine Zusammensetzung zur Anwendung vor oder nach einer Färbung, einer Entfärbung, einer Dauerwelle oder einer Entkrausung oder eine Zusammensetzung zur Anwendung zwischen den beiden Stufen einer Dauerwelle oder einer Entkrausung darstellt.

**23.** Verfahren zur kosmetischen Behandlung von Keratinmaterialien, insbesondere der Haare, **dadurch gekennzeichnet, dass** es darin besteht, dass auf diese Materialien eine Zusammensetzung, wie sie in einem der Ansprüche 1 bis 22 definiert ist, aufgebracht wird und anschließend gegebenenfalls mit Wasser gespült wird.

**24.** Verfahren zur Verbesserung der Lagerstabilität einer kosmetischen Zusammensetzung, die mindestens ein Cyclodextrin und mindestens ein Tensid enthält, das darin besteht, der Zusammensetzung ein verdickendes Acrylpolymer und einen flüssigen Ester einer Carbonsäure mit 4 bis 6 Kohlenstoffatomen mit einem Alkohol mit 12 bis 26 Kohlenstoffatomen zuzugeben.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 457688 A
- WO 9803155 A
- WO 03088934 A
- US 3915921 A
- US 4509949 A
- WO 0031154 A
- EP 750899 A
- US 5089578 A
- FR 2416723
- US 2798053 A
- US 2923692 A
- DE 19625810
- US 2528378 A
- US 2781354 A
- EP 0337354 A
- FR 2270846 A
- FR 2383660 A
- FR 2598611 A
- FR 2470596 A
- FR 2519863 A
- FR 8516334 A
- US 4957732 A
- EP 186507 A
- EP 342834 A
- EP 412704 A
- EP 412707 A
- EP 640105 A
- WO 9500578 A
- EP 582152 A
- WO 9323009 A
- US 4693935 A
- US 4728571 A
- US 4972037 A
- DE 4424530
- DE 4424533
- DE 4402929
- DE 4420736
- WO 9523807 A
- WO 9407844 A
- EP 0646572 A
- WO 9516665 A
- FR 2673179
- EP 0227994 A
- WO 9424097 A
- WO 9410131 A


### Littérature non-brevet citée dans la description


- Self-assembling amphiphilic polyelectrolytes and their nanostructures. *Chinese Journal of Polymer Science,* 2000, vol. 18 (40), 323-336
- Miscelle formation of random copolymers of sodium 2-(acrylamido)-2-methylpropanesulfonate and a non-ionic surfactant macromonomer in water as studied by fluorescence and dynamic light scattering. *Macromolecules,* 2000, vol. 33 (10), 3694-3704
- Solution properties of miscelle networks formed by non-ionic moieties covalently bound to an polyelectrolyte : salt effects on rheological behavior. *Langmuir,* 2000, vol. 16 (12), 5324-5332
- Stimuli responsive amphiphilic copolymers of sodium 2-(acrylamido)-2-methylpropanesulfonate and associative macromonomers. *Polym. Preprint, Div. Polym. Chem.,* 1999, vol. 40 (2), 220-221
- **M.R. PORTER.** Handbook of Surfactants. 1991, 116-178
- **WALTER NOLL.** Chemistry and Technology of Silicones. Academie Press, 1968
- **TODD ; BYERS.** Volatile Silicone fluids for cosmetics. *Cosmetics and toiletries,* Janvier 1976, vol. 91, 27-32